# EUROPÄISCHE PATENTSCHRIFT

(11) **EP 1 489 969 B1**
(45) Veröffentlichungstag und Bekanntmachung des Hinweises auf die Patenterteilung: **31.01.2007**
(21) Anmeldenummer: 03717147.7
(22) Anmeldetag: 13.03.2003
(51) Int. Cl.: A61B 6/03

(54) **COMPUTERTOMOGRAPH MIT ENERGIEDISKRIMINIERENDEN DETEKTOREN**
COMPUTER TOMOGRAPH COMPRISING ENERGY DISCRIMINATING DETECTORS
TOMODENSITOMETRE EQUIPE DE DETECTEURS A DISCRIMINATION D'ENERGIE

(30) Priorität: 21.03.2002 DE 10212638
(43) Veröffentlichungstag der Anmeldung: 29.12.2004
(73) Patentinhaber: SIEMENS AKTIENGESELLSCHAFT, 80333 München (DE)
(72) Erfinder: VON DER HAAR, Thomas, 90482 Nürnberg (DE); HEISMANN, Björn, 91052 Erlangen (DE)
(86) Internationale Anmeldenummer: PCT/DE2003/000818
(87) Internationale Veröffentlichungsnummer: WO 2003/079903

(56) Entgegenhaltungen:
- EP-A- 0 782 375
- DE-A- 10 127 267
- US-A- 4 651 005
- PATENT ABSTRACTS OF JAPAN vol. 2000, no. 10, 17. November 2000 (2000-11-17) -& JP 2000 189409 A (FUJI PHOTO FILM CO LTD), 11. Juli 2000 (2000-07-11)

## Beschreibung

Die vorliegende Erfindung betrifft einen Computertomographen und ein Verfahren zum Nachweis von Röntgenstrahlung mit einer aus einer Vielzahl von Detektoren bestehenden Detektoreinheit.

Bei mannigfaltigen Problemgestaltungen der Medizin werden Untersuchungen mit Hilfe von Computertomographen durchgeführt. Auch in einigen Bereichen des Maschinenbaus, insbesondere der Materialkunde und der Flugsicherheit werden derartige Untersuchungen zu Prüfzwecken eingesetzt.

Dabei wird Röntgenstrahlung verwendet, da diese Festkörper, z.B. nichtmetallische Körper, teilweise zu durchdringen vermag, so dass Erkenntnisse über die Verteilung von Materie innerhalb des betrachteten Körpers gewonnen werden können.

Nachteilig an der Verwendung von Röntgenstrahlung ist, dass diese ab einer gewissen Dosis biologisches Gewebe schädigen kann. Deshalb ist es gerade in der Medizin erstrebenswert, die für eine Messung erforderliche Strahlendosis gering zu halten.

Zum Nachweis von Röntgenstrahlung ist bekannt, dass diese von bestimmten Szintillations-Materialien absorbiert werden kann, wobei die Energie der absorbierten Röntgenquanten in Licht umgewandelt wird. Die Anzahl der erzeugten Photonen pro Röntgenquant ist dabei im allgemeinen etwa proportional zu seiner Quantenenergie. Eine Photodiode wandelt das Licht in einen Strom, der von einem Analog-Digital-Umsetzer digitalisiert wird. Da die Selbstabsorption des Lichts im Szintillations-Material die Lichtausbeute verringert, sind dem Szintillations-Material häufig Moleküle beigemischt, die eine Frequenzverschiebung des erzeugten Lichtes bewirken, um so die Selbstabsorption des erzeugten Lichtes zu vermindern.

Weiter sind zum Nachweis von Röntgenstrahlung auch bestimmte Halbleitermaterialien bekannt, in denen die einfallende Röntgenstrahlung Ladungsträger zu erzeugen vermag. Die Anzahl der erzeugten Ladungsträger pro Röntgenquant ist dabei im allgemeinen etwa proportional zu seiner Quantenenergie.

Die bekannten Detektoren zum Nachweis von Röntgenstrahlung machen sich die vorstehend beschriebenen Effekte zu nutze. Dabei ist zu beachten, dass bei den bekannten integrierenden Detektoren pro Messung lediglich ein Messwert ermittelt wird. Somit werden die von der Vielzahl der pro Messperiode empfangenen Röntgenquanten erzeugten Lichtblitze bzw. Ladungen über die Dauer der Messperiode aufintegriert. Die Intensität der empfangenen Röntgenstrahlung (die Anzahl der empfangenen Röntgenquanten mittlerer Quantenenergie pro Zeiteinheit) ergibt sich dann durch Division des von dem Detektor aufintegrierten Wertes durch die stochastisch zu ermittelnde mittlere Quantenenergie pro Röntgenquant.

Da die in der Computertomographie zu Messzwecken emittierte Mess-Röntgenstrahlung üblicherweise ein polychromatisches Spektrum aufweist, sind in diesem Zusammenhang Aufhärtungseffekte zu berücksichtigen. Bei dem Durchgang der von einer Strahlungsquelle emittierten Mess-Röntgenstrahlung durch ein Messobjekt erfährt die Röntgenstrahlung in Abhängigkeit von der durchdrungenen Materie und der Länge des Strahlenganges durch die Materie hindurch eine z.T. starke Unterdrückung niederenergetischer Anteile ihres Spektrums. Die Streustrahlung wird dadurch ebenso wie die mittlere Quantenenergie der empfangenen Röntgenquanten zu höheren Energien im Spektrum verschoben.

Zum Nachweis der zweidimensionalen Verteilung und somit zum Erstellen eines Bildes der einfallenden Röntgenstrahlung ist es bekannt, eine Vielzahl von gleichartigen Detektoren zu einer Detektoreinheit zum Detektieren einfallender Strahlung und zur Abgabe entsprechender Bildinformationen zusammenzufassen. Die Detektoren sind dabei vorzugsweise in einer Ebene in Form eines Rasters nebeneinander angeordnet.

Dies hat zur Folge, dass sich aufgrund von Aufhärtungseffekten für jeden Detektor einer Detektoreinheit in Abhängigkeit von der Materialverteilung in dem betrachteten Messobjekt ein unterschiedlicher Wert für die tatsächliche mittlere Quantenenergie pro Röntgenquant ergibt. Dieser tatsächliche Wert kann nur näherungsweise mittels stochastischer Methoden bestimmt werden. Insbesondere in Bereichen, in denen unterschiedliche Materialien in dem betrachteten Messobjekt aneinandergrenzen (z.B. Knochenkanten), ist die näherungsweise Bestimmung der mittleren Quantenenergie pro Röntgenquant trotz numerischen Korrekturen stark fehlerbehaftet.

Ein weitere Störgröße bei der Messung von Röntgenstrahlung mittels Computertomographen ist die je nach betrachtetem Messobjekt stärker oder weniger stark ausgeprägte Streustrahlung. Die Streustrahlung kann je nach Spektrum der emittierten Mess-Röntgenstrahlung und Art des betrachteten Messobjektes mehrere zehn Prozent der emittierten Mess-Röntgenstrahlung ausmachen. Sie führt zu einer erheblichen Kontrastverschlechterung des von den Detektoren der Detektoreinheit gewonnenen Messergebnisses.

Deshalb ist vor der Detektoreinheit bekannter Computertomographen ein Streustrahlenraster vorgesehen, durch das nur Röntgenquanten, die eine bestimmte Richtung und Energie haben (und somit für die Messung wichtig sind), hindurchtreten können.

Das Streustrahlenraster weist in der Regel ein spezielles Kollimatorsystem in Form einer Lamellenanordnung auf, so dass auch Röntgenquanten der emittierten Mess-Röntgenstrahlung, die auf die Lamellenwände treffen, absorbiert werden.

Das Vorsehen eines Streustrahlenrasters hat demnach zur Folge, dass einige Prozent der Strahlungsquanten einer zu Messzwecken emittierten Mess-Röntgenstrahlung in dem Streustrahlenraster absorbiert werden, und somit von den Detektoren nicht mehr erfasst werden können.

Folglich muss die Intensität der zu Messzwecken emittierten Strahlung aufgrund des Streustrahlenrasters entsprechend erhöht werden.

Dies führt bei medizinischen Anwendungen unvermeidbar zu einer erhöhten Patientendosis.

Weiter kann die Streustrahlung auch durch das Vorsehen eines Streustrahlenrasters häufig nicht ausreichend gut unterdrückt werden.

Es ist Aufgabe der vorliegenden Erfindung, einen Computertomograph und ein Verfahren zum Nachweis von Röntgenstrahlung mit einer aus einer Vielzahl von Detektoren bestehenden Detektoreinheit zur Verfügung zu stellen, bei dem eine auf Streustrahlungsquanten oder Aufhärtungseffekte zurückzuführende Beeinträchtigung des Messergebnisses einfach und zuverlässig vermieden wird.

Die Aufgabe wird gelöst durch einen Computertomograph, aufweisend:
- eine Strahlungsquelle zum Emittieren von Röntgenstrahlung mit einer vorgegebenen Intensität und einem vorgegebenen Spektrum;
- eine aus einer Vielzahl von Detektoren bestehende Detektoreinheit zum Nachweis von Röntgenstrahlung, wobei die einzelnen Detektoren der Detektoreinheit ausgebildet sind, um einfallende Röntgenquanten der Röntgenstrahlung zu empfangen und die empfangene Röntgenstrahlung hinsichtlich der Anzahl an Röntgenquanten, deren Quantenenergie einen vorgegebenen Schwellenwert überschreitet, zu erfassen;
- eine Übertragungseinrichtung zum Übertragen der von den Detektoren der Detektoreinheit erfassten Informationen an eine Auswerteeinrichtung und
- eine Auswerteeinrichtung, die ausgebildet ist, um anhand der von den Detektoren der Detektoreinheit erfassten Informationen ein Messergebnis von einem Messobjekt, welches von der Röntgenstrahlung durchdrungen wird, zu berechnen, wobei die einzelnen Detektoren der Detektoreinheit ausgebildet sind, um die empfangene Röntgenstrahlung sowohl hinsichtlich ihrer Intensität als auch hinsichtlich der Quantenenergie der einzelnen Röntgenquanten der empfangenen Röntgenstrahlung zu erfassen und pro Messperiode ein Spektrum auszugeben, das neben einer Information über die Anzahl der pro Messperiode empfangenen Röntgenquanten mittlerer Quantenenergie und damit der Intensität auch eine Information über die jeweilige Quantenenergie der Röntgenquanten und damit das Spektrum der empfangenen Röntgenstrahlung enthält; und
dass die Auswerteeinrichtung ferner ausgebildet ist, um das Messergebnis von dem Messobjekt anhand der von den Detektoren erfassten Informationen Intensität und Quantenenergie der einzelnen Röntgenquanten der empfangenen Röntgenstrahlung unter Berücksichtigung der Intensität und des Spektrums der von der Strahlungsquelle emittierten Röntgenstrahlung zu berechnen.

Da die Detektoren der Detektoreinheit des Computertomographen gemäß der vorliegenden Erfindung ausgebildet sind, um einfallende Röntgenstrahlung zu empfangen und die empfangene Röntgenstrahlung sowohl hinsichtlich der Intensität als auch hinsichtlich der Quantenenergie der einzelnen Röntgenquanten der empfangenen Röntgenstrahlung zu erfassen, wird am Ausgang der Detektoren der Detektoreinheit statt eines einzelnen Messwertes pro Messperiode ein Spektrum ausgegeben, das neben einer Information über die Anzahl der pro Messperiode empfangenen Röntgenquanten mittlerer Quantenenergie (Intensität) auch eine Information über die jeweilige Quantenenergie der Röntgenquanten (das Spektrum) der empfangenen Röntgenstrahlung enthält.

Mit einem solchen Aufbau ist es durch Vergleichen der Intensität und des Spektrums der von einer Strahlungsquelle emittierten Röntgenstrahlung mit der von den Detektoren der Detektoreinheit des erfindungsgemäßen Computertomographen erfassten Intensität und dem Spektrum der empfangenen Röntgenstrahlung auf besonders einfache und zuverlässige Weise möglich, ein besonders detailliertes Messergebnis von einem betrachteten Messobjekt zu berechnen.

Anhand der so gewonnenen Information ist es möglich, Einflüsse, die auf Streustrahlung zurückzuführen sind, zusätzlich zu einem ggf. vorhandenen Streustrahlenraster weiter zu unterdrücken.

Weiter ist es durch Betrachtung des erhaltenen Spektrums besonders zuverlässig möglich, Aufhärtungseffekte in der empfangenen Röntgenstrahlung, wie sie z. B. an Knochenkanten auftreten, anhand der Verschiebung des Spektrums der empfangenen Röntgenstrahlung zu detektieren. Die so detektierten Aufhärtungseffekte können dann bei der Weiterverarbeitung der von den Detektoren der Detektoreinheit gewonnenen Information entsprechend berücksichtigt und ggf. korrigiert werden.

Bei der Weiterverarbeitung der von den Detektoren der Detektoreinheit gewonnenen Information ist weiter vorteilhaft, dass eine quantitative Auswertung der von dem erfindungsgemäßen Computertomographen gewonnenen spektralen Daten (beispielsweise durch p-Z Transformation) mit den bei herkömmlichen Computertomographen bekannten Verfahren möglich ist.

Ferner weist die Elektronik der Detektoren des erfindungsgemäßen Computertomographen wesentlich geringere Analogteile als die Elektronik herkömmlich Detektoren auf, da ein Aufintegrieren einer Vielzahl von auf Röntgenquanten der empfangenen Röntgenstrahlung zurückzuführenden Teil-Ereignissen nicht nötig ist. Somit kann die Elektronik des erfindungsgemäßen Computertomographen kleiner, kostengünstiger und störungssicherer bereitgestellt werden.

Zusammenfassend ist es gemäß der vorliegenden Erfindung möglich, einen Computertomograph aufweisend eine aus einer Vielzahl von Detektoren bestehende Detektoreinheit zum Nachweis von Röntgenstrahlung zur Verfügung zu stellen, bei dem eine auf Streustrahlungsquanten oder Aufhärtungseffekte zurückzuführende Beeinträchtigung des Messergebnisses einfach und zuverlässig vermieden wird.

Gemäß eines ersten bevorzugten Ausführungsform weisen die Detektoren der Detektoreinheit eine Vielzahl von parallel geschalteten Vergleichern mit jeweils einem Schwellenwert auf, und ist jedem Vergleicher ein Zähler zugeordnet, wobei die Vergleicher ausgebildet sind, um den jeweils zugehörigen Zähler um eine Einheit zu erhöhen, wenn die Quantenenergie eines Röntgenquants der empfangenen Röntgenstrahlung den Schwellenwert des jeweiligen Vergleichers überschreitet.

Durch einen derartigen Aufbau des Detektors ist es auf besonders einfache Weise möglich, sowohl die Intensität als auch das Spektrum der empfangenen Röntgenstrahlung zu erfassen. Da ferner die Anzahl der empfangenen Röntgenquanten mit einer gewissen Quantenenergie von allen Zählern der Vergleicher mit niedrigeren Schwellwerten mit erfasst werden, werden keine Ereignisse verworfen. Die Anzahl der Röntgenquanten mit einer Quantenenergie innerhalb eines Schwellwertbereichs kann dann einfach aus der Differenz der Zählerstände zweier Vergleicher mit benachbarten Schwellenwerten berechnet werden. Dabei profitiert man von der Korrelation der Zählraten bei Zählern, so dass der statistische Fehler bei der Subtraktion nicht ansteigt.

Vorzugsweise sind die Schwellenwerte der Vergleicher frei einstellbar, so dass der erfindungsgemäße Computertomograph an verschiedene zu betrachtende Messobjekte und verschieden Messverfahren angepasst werden kann.

Die von den Detektoren der Detektoreinheit gewonnenen Informationen lassen sich besonders einfach weiterverarbeiten, wenn die Detektoren der Detektoreinheit eine Vielzahl von Pulslogiken aufweisen. Die Pulslogiken bewirken eine zeitliche Normierung der Ausgangssignale der Vergleicher. Dabei ist jeweils eine Pulslogik den jeweiligen Vergleichern nachgeschaltet und den jeweiligen Zählern vorgeschaltet.

Bevorzugt weisen die Detektoren der Detektoreinheit eine Empfangsfläche für die Röntgenstrahlung auf, die aus Gadoliniumoxisulfid-Keramik, Bismuth-Germanium-Oxid oder Lutetium-Oxyorthosilikat gebildet ist. Diese sehr schnellen Szintillator-Materialien ermöglichen die in dem erfindungsgemäßen Computertomographen vorzugsweise verwendete Zählrate von bis zu 10MHz bei Pixelgrößen von etwa 1/50mm².

Alternativ können die Detektoren jedoch auch eine direktwandelnde Empfangsfläche für die Röntgenstrahlung aufweisen, die vorzugsweise aus Cadmium-Zink-Tellurid oder Cadmium-Tellurid gebildet ist.

Der Vorteil direktwandelnder Detektoren ist insbesondere darin zu sehen, dass ein großer Teil einer für die Weiterverarbeitung eines von dem Detektor erzeugten Signals erforderlichen Auswertelektronik in die Detektoren integriert werden kann, so dass die Komplexität der Detektoreinheit nicht zuletzt aufgrund der Reduzierung der Anzahl der herauszuführenden Leitungen reduziert werden kann.

Die der vorliegenden Erfindung zugrundeliegende Aufgabe wird auch von einem Verfahren zum Nachweis von Röntgenstrahlung durch einen eine aus einer Vielzahl von Detektoren bestehende Detektoreinheit aufweisenden Computertomographen gelöst, aufweisend die folgenden Schritte:
- Erfassen der mittels der einzelnen Detektoren der Detektoreinheit empfangenen Röntgenstrahlung hinsichtlich der Anzahl an Röntgenquanten, deren Quantenenergie einen vorgegebenen Schwellenwert überschreitet,
- Übertragung der mittels der Detektoren der Detektoreinheit erfassten Informationen an eine Auswerteeinrichtung, und
- Berechnung eines Messergebnisses von einem Messobjekt, welches von der Röntgenstrahlung durchdrungen wird, mittels der Auswerteeinrichtung anhand der von den Detektoren der Detektoreinheit erfassten Informationen, wobei die mittels der einzelnen Detektoren der Detektoreinheit empfangene Röntgenstrahlung sowohl hinsichtlich ihrer Intensität als auch hinsichtlich der Quantenenergie der einzelnen Röntgenquanten der empfangenen Röntgenstrahlung erfasst wird, dass durch die einzelnen Detektoren der Detektoreinheit pro Messperiode ein Spektrum ausgegeben wird, das neben einer Information über die Anzahl der pro Messperiode empfangenen Röntgenquanten mittlerer Quantenenergie und damit der Intensität auch eine Information über die jeweilige Quantenenergie der Röntgenquanten und damit das Spektrum der empfangenen Röntgenstrahlung enthält, und dass die Berechnung des Messergebnisses von dem Messobjekt mittels der Auswerteeinrichtung anhand der von den Detektoren erfassten Informationen Intensität und Quantenenergie der einzelnen Röntgenquanten der empfangenen Röntgenstrahlung unter Berücksichtung der Intensität und des Spektrums der von einer Strahlungsquelle emittierten Röntgenstrahlung erfolgt.

Mit einem solchen Verfahren ist es durch Vergleichen der Intensität und des Spektrums der von einer Strahlungsquelle emittierten Röntgenstrahlung mit der von den Detektoren der Detektoreinheit erfassten Intensität und dem Spektrum der empfangenen Röntgenstrahlung auf besonders einfache und zuverlässige Weise möglich, Streustrahlungseinflüsse und Aushärtungseffekte zu korrigieren und so ein besonders detailliertes Messergebnis von einem betrachteten Messobjekt zu berechnen.

Gemäß einer ersten Ausführungsform des erfindungsgemäßen Verfahrens weist der Schritt der Erfassung der mittels des Detektors der Detektoreinheit empfangenen Röntgenquanten die folgenden Schritte auf:
- Detektion eines in dem Detektor in Folge eines empfangenen Röntgenquants erzeugten Signals, dessen Signalhöhe proportional zur Quantenenergie des empfangenen Röntgenquants ist;
- Vergleich der Signalhöhe mit einer Vielzahl von vorgegebenen Schwellenwerten;
- Erhöhung eines jeweils einem Bereich zwischen zwei benachbarten Schwellenwerten zugeordneten Zählers um eine Einheit, wenn die Signalhöhe des Signals in dem Bereich zwischen den beiden benachbarten Schwellenwerten liegt.

Da die Zählerstände der Zähler am Ende einer Messperiode somit sowohl eine Information über die Anzahl an empfangenen Röntgenquanten als auch über die jeweilige Quantenenergie der empfangenen Röntgenquanten erhalten, ist es ein leichtes, anhand der Zählerständen der Zähler sowohl die Intensität als auch das Spektrum der empfangenen Röntgenstrahlung anzugeben.

Gemäß einer alternativen zweiten besonders bevorzugten Ausführungsform des erfindungsgemäßen Verfahrens weist der Schritt der Erfassung der mittels des Detektors der Detektoreinheit empfangenen Röntgenquanten die folgenden Schritte auf:
- Detektion eines in dem Detektor in Folge eines empfangenen Röntgenquants erzeugten Signals, dessen Signalhöhe proportional zur Quantenenergie des empfangenen Röntgenquants ist;
- Vergleich der Signalhöhe mit einer Vielzahl von vorgegebenen Schwellenwerten;
- Erhöhung von Zählern, die jeweils einem Schwellenwert zugeordnet sind, um eine Einheit, wenn die Signalhöhe des Signals den jeweiligen Schwellenwert überschreitet.

Besonders vorteilhaft an dieser Vorgehensweise ist, dass keine Ereignisse verworfen werden, da die Anzahl der empfangenen Röntgenquanten mit einer gewissen Quantenenergie von allen Zählern mit niedrigerem Schwellwert mit erfasst wird. Die Anzahl der Röntgenquanten mit einer Quantenenergie innerhalb eines Schwellwertbereichs kann dann einfach aus der Differenz der Zählerstände der Zähler zweier Vergleicher mit benachbarten Schwellenwerten berechnet werden.

Weiter ist es vorteilhaft, wenn ein in dem Detektor in Folge eines empfangenen Röntgenquants erzeugtes Signal verworfen wird, wenn die ermittelte Signalhöhe des Signals kleiner als ein niedrigster Schwellenwert ist.

Auch bei dem erfindungsgemäßen Verfahren ist es besonders vorteilhaft, wenn die Schwellenwerte frei einstellbar sind.

Im Folgenden werden bevorzugte Ausführungsformen der vorliegenden Erfindung unter Zuhilfenahme von Figuren beschrieben. In den Figuren sind gleiche Elemente mit den gleichen Bezugszeichen versehen. Dabei zeigen
- Fig. 1: eine aus einer Vielzahl von Detektoren bestehende Detektoreinheit eines Computertomographen zum Nachweis von Röntgenstrahlung,
- Fig. 2: schematisch wesentliche Elemente eines Detektors des erfindungsgemäßen Computertomographen gemäß einer besonders bevorzugten Ausführungsform,
- Fig. 3: eine prinzipielle Weiterbildung des Detektors von Fig. 2,
- Fig. 4: schematisch wesentliche Elemente eines bevorzugten Messaufbaus mit dem erfindungsgemäßen Computertomographen, und
- Fig. 5: ein Ablaufdiagramm einer besonders bevorzugten Ausführungsform des erfindungsgemäßen Verfahrens zum Nachweis von Röntgenstrahlung mit einem eine aus einer Vielzahl von Detektoren bestehende Detektoreinheit aufweisenden Computertomographen.

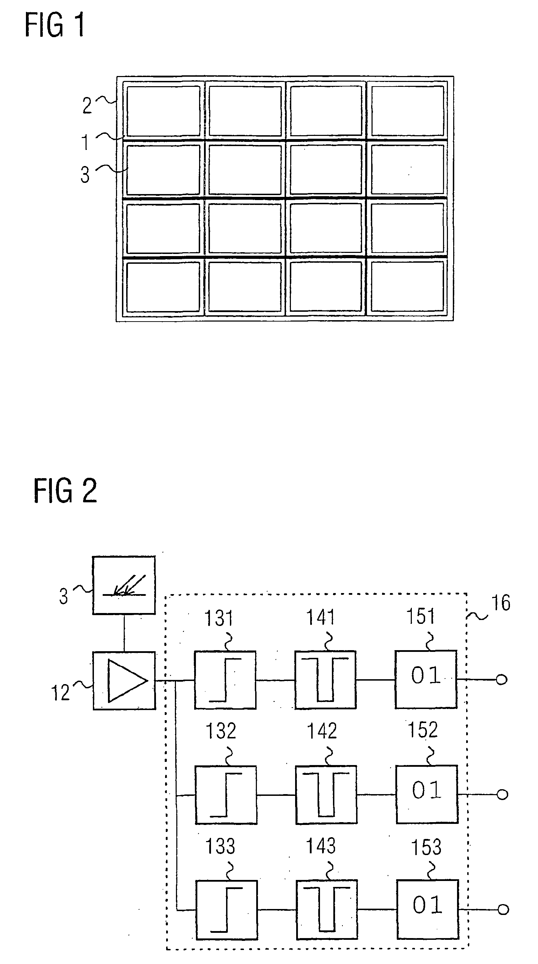

In Fig. 1 ist eine aus einer Vielzahl von Detektoren bestehende Detektoreinheit eines Computertomographen zum Nachweis von Röntgenstrahlung gezeigt.

Die einzelnen Detektoren 1 der Detektoreinheit 2 haben jeweils den gleichen Aufbau und weisen jeweils eine Empfangsfläche 3 für Röntgenstrahlung auf.

In der gezeigten bevorzugten Ausführungsform weisen die Empfangsflächen 3 der Detektoren ein Szintillator-Material, in /3 dem einfallende Röntgenquanten in Licht umgewandelt werden, auf. Dabei ist die Zahl der von einem empfangenen Röntgenquant erzeugten Photonen in etwa proportional zu der Quantenenergie des empfangenen Röntgenquants. Als Szintillator-Material findet in Fig. 1 Bismuth-Germanium-Oxid (Bi₄Ge₃O₁₂) Verwendung. Alternativ sind jedoch auch Gadoliniumoxisulfid (Gd₂O₂S)-Keramik oder Lutetium-Oxyorthosilikat (Lu₂SiO₅) aufgrund der Schnelligkeit dieser Szintillator-Materialien sehr gut geeignet.

Alternativ können die Empfangsflächen 3 der Detektoren jedoch auch aus Cadmium-Zink-Tellurid (CdZnTe) oder Cadmium-Tellurid (CdTe) aufgebaut sein, da diese Materialien in Folge eines empfangenen Röntgenquants direkt (d.h. ohne den Umweg über Licht) ein elektrisches Signal ausgeben können. Der Wert / die Höhe des Signals (in Form einer erzeugten Ladung oder Spannung oder eines erzeugten Stroms) ist dabei in etwa proportional zu der Quantenenergie des empfangenen Röntgenquants. Vorteilhaft an direkt wandelnden Detektoren ist insbesondere, dass einen Teil einer (nicht gezeigten) weiterverarbeitenden Auswertelektronik für die Detektoren direkt in den jeweiligen Detektor integriert werden kann.

In Fig. 2 sind schematisch wesentliche Elemente eines Detektors des erfindungsgemäßen Computertomographen gemäß einer besonders bevorzugten Ausführungsform gezeigt.

Wie vorstehend erläutert wird in der Empfangsfläche 3 des in Fig. 2 gezeigten Detektors 1 in Folge eines empfangenen Röntgenquants ein Signal erzeugt, dessen Signalhöhe proportional zu der Quantenenergie des empfangenen Röntgenquants ist. Dieses Signal wird von einem Verstärker 12 verstärkt.

An den Verstärker 12 ist eine Erfassungsschaltung 16 angeschlossen, die drei parallel geschaltete Vergleicher 131, 132 und 133 aufweist.

Jedem der parallel geschalteten Vergleicher 131, 132 und 133 ist ein anderer frei einstellbarer Schwellenwert zugewiesen. In dem gezeigten Beispiel ist dem Vergleicher 131 der niedrigste und dem Vergleicher 133 der höchste Schwellenwert zugewiesen.

Die Vergleicher 131, 132 und 133 sind ausgebildet, um das von dem Verstärker 12 ausgegebene Signal mit ihrem jeweiligen Schwellenwert zu vergleichen und ein positives Signal auszugeben, wenn das von dem Verstärker 12 empfangene Signal höher als der jeweilige Schwellenwert ist.

In Serie mit den Vergleichern 131, 132 und 133 ist jeweils eine Pulslogik 141, 142 und 143 geschaltet. Die Pulslogik 141, 142 und 143 ist jeweils ausgebildet, um ein zeitliche Normierung der Ausgangssignale der Vergleicher 131, 132 und 133 zu bewirken. Weiter ist in Serie zu der Pulslogik 141, 142 und 143 jeweils ein Zähler 151, 152 und 153 geschaltet.

Infolge eines von dem jeweiligen Vergleicher 131, 132 und 133 ausgegebenen und von der jeweiligen Pulslogik 141, 142 und 143 normierten positiven Signals wird der jeweilige Zähler 151, 152 und 153 um eine Einheit erhöht.

Dabei sind die Pulslogiken 141, 142 und 143 vorzugsweise untereinander synchronisiert und weisen eine nicht gezeigte gemeinsame Steuerleitung auf.

Wird bei der in Fig. 2 gezeigten besonders bevorzugten Ausführungsform eines Detektors des erfindungsgemäßen Computertomographen somit ein Röntgenquant empfangen, dessen Quantenenergie über dem Schwellenwert des Vergleichers 132 und somit auch über dem Schwellenwert des Vergleichers 131, aber unter dem Schwellenwert des Vergleichers 133 liegt, so geben sowohl der Vergleicher 131 als auch der Vergleicher 132 ein positives Ausgangssignal aus. Infolge dessen werden die Zähler 151 und 152 um eins erhöht. Der Vergleicher 133 gibt hingegen ein negatives Ausgangssignal aus, und der dem Vergleicher 133 zugehörige Zähler 153 bleibt unverändert.

Wird in dem gezeigten Beispiel ein Röntgenquant empfangen, dessen Quantenenergie über dem Schwellenwert des Vergleichers 131 aber unter dem Schwellenwert des Vergleichers 132 und somit auch unter dem Schwellenwert des Vergleichers 133 liegt, so wird entsprechend nur der Zähler 151 um eins erhöht, wohingegen die Zähler 152 und 153 unverändert bleiben.

Wird hingegen ein Röntgenquant empfangen, dessen Quantenenergie unter dem Schwellenwert des Vergleichers 131 liegt, so wird das Röntgenquant von keinem der Zähler 151, 152 und 153 erfasst.

Somit ist es durch geschickte Wahl des niedrigsten Schwellenwertes möglich, Streustrahlungseinschüsse von vornherein auszuschließen, da diese von keinem Zähler erfasst werden.

Wie aus den vorgezeigten Beispielen leicht deutlich wird, lässt sich die Zahl der empfangenen Röntgenquanten, deren Quantenenergie einem jeweiligen Schwellenwertbereich entspricht, einfach durch Differenz der Zählerstände der Zähler von Vergleichern benachbarter Schwellenwerte berechnen.

Die in Fig. 2 gezeigte besonders bevorzugte Ausführungsform ermöglicht der besseren Übersichtlichkeit wegen lediglich eine spektrale Unterscheidung von Röntgenquanten in vier Quantenenergie-Bereiche (unter dem Schwellenwert des Vergleichers 131, zwischen den Schwellenwerten der Vergleicher 131 und 132, zwischen den Schwellenwerten der Vergleicher 132 und 133 und über dem Schwellenwert des Vergleichers 133).

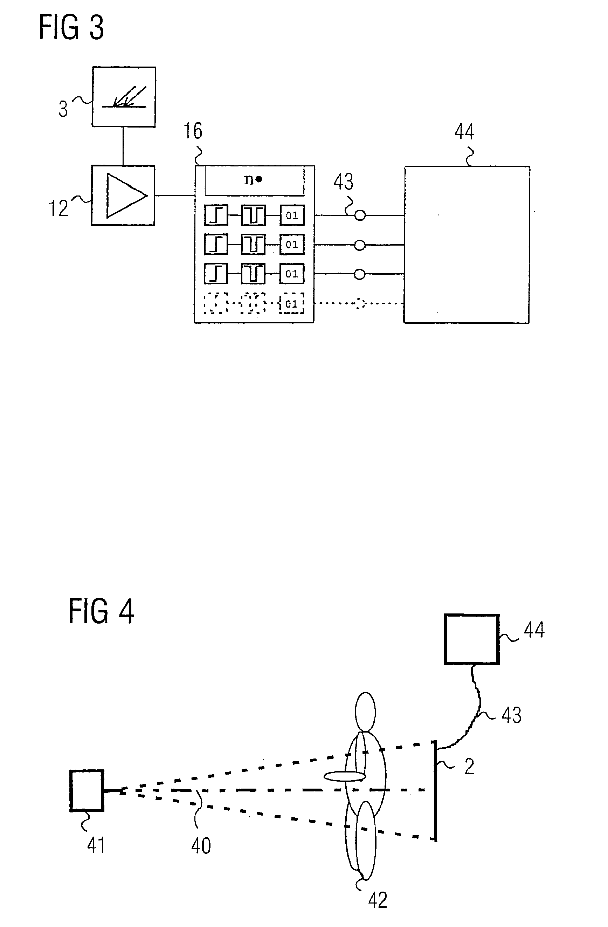

Um ausgehend hiervon eine in der Praxis wünschenswerte höhere spektrale Auflösung der empfangenen Röntgenstrahlung zu erreichen, ist es lediglich erforderlich, in der Erfassungsschaltung 16 eine größere Anzahl von parallel geschalteten Vergleichern mit unterschiedlichen Schwellenwerten vorzusehen. Wie in Fig. 3 angedeutet, ist jedem Vergleicher wiederum eine Pulslogik sowie ein Zähler zuzuordnen. Folglich lässt sich so auf einfache Weise eine nahezu beliebig feine spektrale Auflösung der von der Empfangsfläche 3 des Detektors 1 empfangenen Röntgenstrahlung erreichen.

Somit wird mit dem vorstehend beschriebenen Detektor 1 der Detektoreinheit 2 des erfindungsgemäßen Computertomographen die empfangene Röntgenstrahlung sowohl hinsichtlich der Intensität als auch hinsichtlich der Quantenenergie der einzelnen Röntgenquanten der empfangenen Röntgenstrahlung erfasst.

Gemäß einer in Fig. 4 gezeigten besonders bevorzugten Ausführungsform ist es weiter besonders vorteilhaft, wenn der erfindungsgemäße Computertomograph neben einer Strahlungsquelle 41 zum Emittieren von Röntgenstrahlung 40 mit einer vorgegebenen Intensität und einem vorgegebenen Spektrum eine Übertragungseinrichtung 43 zum Übertragen der von den Detektoren 1 der Detektoreinheit 2 erfassten Informationen an eine Auswerteeinrichtung 44 aufweist.

Dabei ist die Auswerteinrichtung 44 vorzugsweise ausgebildet, um anhand der von den Detektoren 1 der Detektoreinheit 2 erfassten Informationen unter Berücksichtigung der Intensität und des Spektrums der von der Strahlungsquelle 41 emittierten Röntgenstrahlung 40 ein Messergebnis von einem Messobjekt 42, welches von der Röntgenstrahlung 40 der Strahlungsquelle 41 durchdrungen wird, zu berechnen.

Mit diesem Aufbau kann ein besonders genaues und fehlerfreies Messergebnis erhalten werden, da Streustrahlungseinflüsse sowie Aushärtungseffekte wirkungsvoll detektiert, quantifiziert und somit auch korrigiert werden können.

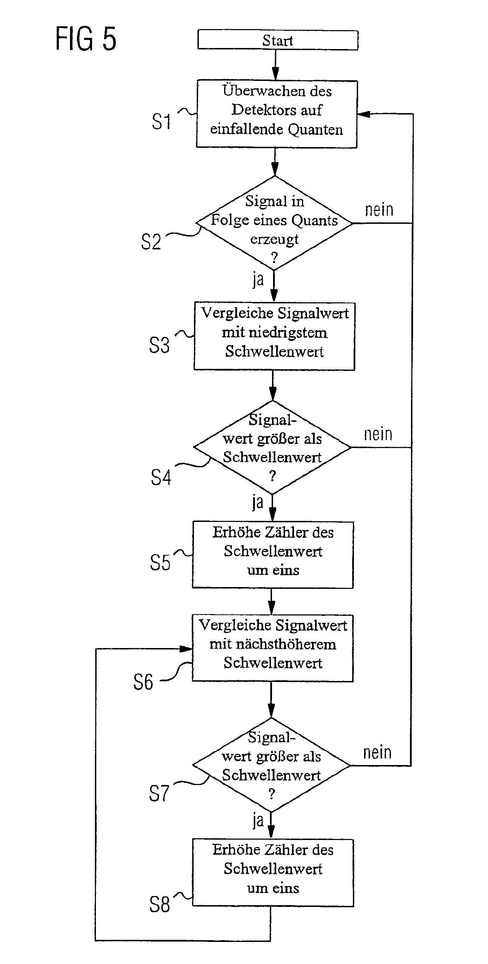

Unter Bezugname auf Fig. 5 wird im Folgenden anhand eines Flussdiagramms eine bevorzugte Ausführungsform des erfindungsgemäßen Verfahrens zum Nachweis von Röntgenstrahlung mit einem eine aus einer Vielzahl von Detektoren 1 bestehende Detektoreinheit 2 aufweisenden Computertomographen beschrieben.

Erfindungsgemäß wird bei dem Verfahren die mittels eines Detektors 1 der Detektoreinrichtung 2 empfangene Röntgenstrahlung 40 sowohl hinsichtlich der Intensität als auch hinsichtlich der Quantenenergie eines einzelnen Röntgenquants der empfangenen Röntgenstrahlung 40 erfasst.

Gemäß der in Fig.5 gezeigten bevorzugten Ausführungsform weist der Schritt des Erfassens der mittels eines jeweiligen Detektors 1 der Detektoreinheit 2 empfangenen Röntgenquanten die folgenden Schritte auf:

In einem ersten Schritt S1 wird der Detektor 1 kontinuierlich auf einfallende Röntgenquanten überwacht, um ein von dem Detektor 1 infolge eines empfangenen Röntgenquants ausgegebenes Analog-Signal zu detektieren. Dabei ist der Detektor 1 so aufgebaut, dass der Wert (die Höhe) des ausgegebenen Signals proportional zu der Quantenenergie des empfangenen Röntgenquants ist (wie es beispielsweise bei Szintillations-Detektoren der Fall ist). Bei diesem ausgegebenen Signal kann es sich beispielsweise um einen elektrischen Strom oder eine Spannung oder Ladung mit einer gewissen Höhe handeln.

Wird in Schritt S2 ein infolge eines empfangenen Röntgenquants von dem Detektor 1 erzeugtes Signal detektiert, so wird der Wert des erzeugten Signals zum Feststellen der Quantenenergie des das Signal verursachenden empfangenen Röntgenquants in Schritt S3 zunächst mit einem ersten, niedrigsten Schwellenwert verglichen.

Wird in Schritt S4 entschieden, dass der Wert des Signals größer als der niedrigste Schwellenwert ist, so wird ein dem niedrigsten Schwellenwert zugehöriger Zähler 151 im folgenden Schritt S5 um eine Einheit erhöht.

Andernfalls kehrt das Verfahren zu Schritt S1 zurück, in dem der Detektor 1 kontinuierlich auf einfallende Röntgenquanten überwacht wird.

Wurde in Schritt S4 entschieden, dass der Wert des Signals größer als der niedrigste Schwellenwert ist, so wird das Signal nach dem Erhöhen des dem niedrigsten Schwellenwert zugeordneten Zählers 151 (siehe Schritt S5) in Schritt S6 mit dem nächsthöheren Schwellenwert verglichen.

Wird im folgendenden Schritt S7 entschieden, dass der Wert des Signals größer als dieser nächsthöhere Schwellenwert ist, so wird auch der diesem Schwellenwert zugehörige Zähler 152, 153 in Schritt S8 inkrementiert.

Anschließend wird das Signal wieder in Schritt S6 mit dem jeweils nächsthöheren Schwellenwert verglichen.

Wird in Schritt S7 entschieden, dass der Signalwert kleiner als der jeweilige Schwellenwert ist, so kehrt das Verfahren zu Schritt S1 zurück, in dem der Detektor 1 kontinuierlich auf einfallende Röntgenquanten überwacht wird.

Es ist zu beachten, dass einzelne Schritte des in Verbindung mit Fig. 5 beschriebene Verfahrens (insbesondere die Schritte S3, S4, S5 und S6, S7, S8), wenn sie von der elektronischen Erfassungsschaltung nach Fig. 2 durchgeführt werden, vorzugsweise nicht - wie in Fig. 5 gezeigt - seriell, sondern parallel verarbeitet werden. Dabei ist eine Taktung der Schritte in Fig. 2 von der Pulslogik vorgegeben und beträgt vorzugsweise einige MHz.

Wie aus der anhand Fig. 5 erläuterten Ausführungsform des erfindungsgemäßen Verfahrens deutlich wird, wird ein von dem Detektor 1 infolge eines empfangenen Röntgenquants erzeugtes Signal verworfen, wenn der Signalwert kleiner als der niedrigste Schwellenwert ist. Damit ist es möglich, durch geeignete Wahl des niedrigsten Schwellenwertes Streustrahlungseinflüsse weitgehend auszuschließen.

Dabei sind die Schwellenwerte jedoch prinzipiell frei einstellbar, so dass auch ein niedrigster Schwellenwert von Null oder nahe Null denkbar ist. Ein solch niedriger Schwellenwert hat den Vorteil, dass kein Ereignis verworfen wird.

Nach Ablauf des vorstehend beschriebenen Verfahrens lässt sich die Anzahl der eingefallenen Röntgenquanten mit einer Quantenenergie, die einem bestimmten Schwellenbereich entspricht, leicht durch Differenz der Zählerstände der benachbarten Schwellenwerten zugeordneten Zähler bestimmen.

In der beschriebenen besonders bevorzugten Ausführungsform läuft das in Fig. 5 dargestellte Verfahren in einer Erfassungsschaltung 16 ab, die in jeden Detektor 1 der Detektoreinheit 2 des erfindungsgemäßen Computertomographen integriert ist.

Gemäß einer nicht eigens dargestellten alternativen Ausführungsform des erfindungsgemäßen Verfahrens kann im Gegensatz zu der vorstehend beschriebenen Ausführungsform auch nur jeweils derjenige Zähler um eine Einheit erhöht werden, der jeweils einem Bereich zwischen zwei benachbarten Schwellenwerten zugeordnet ist, wohingegen die anderen Zähler konstant bleiben. Dies ermöglicht ohne weitere Berechnungen eine direkte Ausgabe der Anzahl der eingefallenen Röntgenquanten mit einer einem gewissen Schwellenbereich zugeordneten Quantenenergie.

Schaltungstechnisch realisieren lässt sich diese alternative Ausführungsform des erfindungsgemäßen Verfahrens besonders einfach beispielsweise dadurch, dass jedem Zähler ein UND-Gatter mit einem invertierenden Eingang vorgeschaltet ist. In diesem Fall sind die Ausgänge der Vergleicher benachbarter Schwellenwerte (ggf. über eine Pulslogik) mit den Eingängen dieses UND-Gatters zu verbinden.

Auch bei dem erfindungsgemäßen Verfahren ist es besonders vorteilhaft, wenn das Verfahren zudem die Schritte einer Übertragung der mittels der Detektoren 1 gewonnenen Information an eine Auswerteeinrichtung 44 und einer Berechnung eines Messergebnisses von einem Messobjekt 42, welches von der Röntgenstrahlung 40 durchdrungen wird, mittels der Auswerteeinrichtung 44 umfasst. Dabei erfolgt die Berechnung des Messergebnisses durch die Auswerteeinrichtung 44 anhand der von den Detektoren 1 erfassten Information unter Berücksichtung der Intensität und des Spektrums der von einer Strahlungsquelle 41 emittierten Röntgenstrahlung 40. Somit können bei der Berechnung des Messergebnisses des Messobjektes 42 neben den Streustrahlungseinflüssen auch Aufhärtungseinflüsse mit hoher Fehlersicherheit korrigiert werden.

Zusammenfassend ist es gemäß der vorliegenden Erfindung durch die Erfassung von sowohl Intensität als auch Spektrum von einer mit einem Detektor 1 einer Detektoreinheit 2 eines Computertomographen empfangenen Röntgenstrahlung 40 möglich, einen Computertomograph und ein Verfahren zum Nachweis von Röntgenstrahlung 40 mit einer aus einer Vielzahl von Detektoren 1 bestehenden Detektoreinheit 2 zur Verfügung zu stellen, bei dem eine auf Streustrahlungsquanten oder Aufhärtungseffekte zurückzuführende Beeinträchtigung des Messergebnisses einfach und zuverlässig vermieden wird.

## Patentansprüche

1. Computertomograph, aufweisend:
- eine Strahlungsquelle (41) zum Emittieren von Röntgenstrahlung (40) mit einer vorgegebenen Intensität und einem vorgegebenen Spektrum;
- eine aus einer Vielzahl von Detektoren (1) bestehende Detektoreinheit (2) zum Nachweis von Röntgenstrahlung (40), wobei die einzelnen Detektoren (1) der Detektoreinheit (2) ausgebildet sind, um einfallende Röntgenquanten der Röntgenstrahlung (40) zu empfangen und die empfangene Röntgenstrahlung (40) hinsichtlich der Anzahl an Röntgenquanten, deren Quantenenergie einen vorgegebenen Schwellenwert überschreitet, zu erfassen;
- eine Übertragungseinrichtung (43) zum Übertragen der von den Detektoren (1) der Detektoreinheit (2) erfassten Informationen an eine Auswerteeinrichtung (44); und
- eine Auswerteeinrichtung (44), die ausgebildet ist, um anhand der von den Detektoren (1) der Detektoreinheit (2) erfassten Informationen ein Messergebnis von einem Messobjekt (42), welches von der Röntgenstrahlung (40) durchdrungen wird, zu berechnen;
**dadurch gekennzeichnet,**
**dass** die einzelnen Detektoren (1) der Detektoreinheit (2) ausgebildet sind, um die empfangene Röntgenstrahlung (40) sowohl hinsichtlich ihrer Intensität als auch hinsichtlich der Quantenenergie der einzelnen Röntgenquanten der empfangenen Röntgenstrahlung (40) zu erfassen und pro Messperiode ein Spektrum auszugeben, das neben einer Information über die Anzahl der pro Messperiode empfangenen Röntgenquanten mittlerer Quantenenergie und damit der Intensität auch eine Information über die jeweilige Quantenenergie der Röntgenquanten und damit das Spektrum der empfangenen Röntgenstrahlung enthält; und
**dass** die Auswerteeinrichtung (44) ferner ausgebildet ist, um das Messergebnis von dem Messobjekt (42) anhand der von den Detektoren (1) erfassten Informationen Intensität und Quantenenergie der einzelnen Röntgenquanten der empfangenen Röntgenstrahlung (40) unter Berücksichtigung der Intensität und des Spektrums der von der Strahlungsquelle (41) emittierten Röntgenstrahlung (40) zu berechnen.

2. Computertomograph nach Anspruch 1,
**dadurch gekennzeichnet,**
**dass** die Detektoren (1) der Detektoreinheit (2) eine Vielzahl von parallel geschalteten Vergleichern (131, 132, 133) mit jeweils einem Schwellenwert aufweisen, und
**dass** jedem Vergleicher (131, 132, 133) ein Zähler (151, 152, 153) zugeordnet ist, und die Vergleicher (131, 132, 133) ausgebildet sind, um den jeweils zugehörigen Zähler (151, 152, 153) um eine Einheit zu erhöhen, wenn die Quantenenergie eines Röntgenquants der empfangenen Röntgenstrahlung (40) den Schwellenwert des jeweiligen Vergleichers (131, 132, 133) überschreitet.

3. Computertomograph nach Anspruch 2,
**dadurch gekennzeichnet,**
**dass** die Schwellenwerte der Vergleicher (131, 132, 133) frei einstellbar sind.

4. Computertomograph nach Anspruch 2 oder 3,
**dadurch gekennzeichnet,**
**dass** die Detektoren (1) der Detektoreinheit (2) eine Vielzahl von Pulslogiken (141, 142, 143) aufweisen, wobei jeweils eine Pulslogik (141, 142, 143) den jeweiligen Vergleichern (131, 132, 133) nachgeschaltet und den jeweiligen Zählern (151, 152, 153) vorgeschaltet ist, und die Pulslogiken (141, 142, 143) eine zeitliche Normierung der Ausgangssignale der Vergleicher (131, 132, 133) bewirken.

5. Computertomograph nach einem der vorherigen Ansprüche,
**dadurch gekennzeichnet,**
**dass** die Detektoren (1) der Detektoreinheit (2) eine Empfangsfläche (3) für die Röntgenstrahlung (40) aufweisen, die aus Gadoliniumoxisulfid-Keramik, Bismuth-Germanium-Oxid oder Lutetium-Oxyorthosilikat gebildet ist.

6. Computertomograph nach einem der Ansprüche 1 bis 4,
**dadurch gekennzeichnet,**
**dass** die Detektoren (1) der Detektoreinheit (2) eine direktwandelnde Empfangsfläche (3) für die Röntgenstrahlung (40) aufweisen, die aus Cadmium-Zinc-Tellurid oder Cadmium-Tellurid gebildet ist.

7. Verfahren zum Nachweis von Röntgenstrahlung durch einen eine aus einer Vielzahl von Detektoren (1) bestehende Detektoreinheit (2) aufweisenden Computertomographen, aufweisend die folgenden Schritte:
- Erfassen der mittels der einzelnen Detektoren (1) der Detektoreinheit (2) empfangenen Röntgenstrahlung (40) hinsichtlich der Anzahl an Röntgenquanten, deren Quantenenergie einen vorgegebenen Schwellenwert überschreitet;
- Übertragung der mittels der Detektoren (1) der Detektoreinheit (2) erfassten Informationen an eine Auswerteeinrichtung (44); und
- Berechnung eines Messergebnisses von einem Messobjekt (42), welches von der Röntgenstrahlung (40) durchdrungen wird, mittels der Auswerteeinrichtung (44) anhand der von den Detektoren (1) der Detektoreinheit (2) erfassten Informationen;
**dadurch gekennzeichnet,**
**dass** die mittels der einzelnen Detektoren (1) der Detektoreinheit (2) empfangene Röntgenstrahlung (40) sowohl hinsichtlich ihrer Intensität als auch hinsichtlich der Quantenenergie der einzelnen Röntgenquanten der empfangenen Röntgenstrahlung (40) erfasst wird,
**dass** durch die einzelnen Detektoren (1) der Detektoreinheit (2) pro Messperiode ein Spektrum ausgegeben wird, das neben einer Information über die Anzahl der pro Messperiode empfangenen Röntgenquanten mittlerer Quantenenergie und damit der Intensität auch eine Information über die jeweilige Quantenenergie der Röntgenquanten und damit das Spektrum der empfangenen Röntgenstrahlung enthält, und
**dass** die Berechnung des Messergebnisses von dem Messobjekt (42) mittels der Auswerteeinrichtung (44) anhand der von den Detektoren (1) erfassten Informationen Intensität und Quantenenergie der einzelnen Röntgenquanten der empfangenen Röntgenstrahlung (40) unter Berücksichtung der Intensität und des Spektrums der von einer Strahlungsquelle (41) emittierten Röntgenstrahlung (40) erfolgt.

8. Verfahren zum Nachweis von Strahlung nach Anspruch 7,
**dadurch gekennzeichnet,**
**dass** die Erfassung der mittels des Detektors (1) der Detektoreinheit (2) empfangenen Röntgenquanten die folgenden Schritte aufweist:
- Detektion eines in dem Detektor (1) in Folge eines empfangenen Röntgenquants erzeugten Signals, dessen Signalhöhe proportional zur Quantenenergie des empfangenen Röntgenquants ist;
- Vergleich der Signalhöhe mit einer Vielzahl von vorgegebenen Schwellenwerten;
- Erhöhung eines jeweils einem Bereich zwischen zwei benachbarten Schwellenwerten zugeordneten Zählers (151, 152, 153) um eine Einheit, wenn die Signalhöhe des Signals in dem Bereich zwischen den beiden benachbarten Schwellenwerten liegt.

9. Verfahren zum Nachweis von Strahlung nach Anspruch 7,
**dadurch gekennzeichnet,**
**dass** die Erfassung der mittels des Detektors (1) der Detektoreinheit (4) empfangenen Röntgenquanten die folgenden Schritte aufweist:
- Detektion eines in dem Detektor (1) in Folge eines empfangenen Röntgenquants erzeugten Signals, dessen Signalhöhe proportional zur Quantenenergie des empfangenen Röntgenquants ist;
- Vergleich der Signalhöhe mit einer Vielzahl von vorgegebenen Schwellenwerten;
- Erhöhung von Zählern (151, 152, 153), die jeweils einem Schwellenwert zugeordnet sind, um eine Einheit, wenn die Signalhöhe des Signals den jeweiligen Schwellenwert überschreitet.

10. Verfahren zum Nachweis von Strahlung nach Anspruch 8 oder 9,
**dadurch gekennzeichnet,**
**dass** ein in dem Detektor (1) in Folge eines empfangenen Röntgenquants erzeugtes Signal verworfen wird, wenn die ermittelte Signalhöhe des Signals kleiner als ein niedrigster Schwellenwert ist.

11. Verfahren zum Nachweis von Strahlung nach Anspruch 8, 9 oder 10,
**dadurch gekennzeichnet,**
**dass** die Schwellenwerte frei einstellbar sind.

## Claims

1. Computer tomograph having:
- a radiation source (41) for emission of X-ray radiation (40) with a predetermined intensity and a predetermined spectrum;
- a detector unit (2), which comprises a large number of detectors (1), for verification of X-ray radiation (40), with the individual detectors (1) in the detector unit (2) being designed to receive incident X-ray quanta in the X-ray radiation (40) and to detect the number of X-ray quanta in the received X-ray radiation (40) whose quantum energy exceeds a predetermined threshold value;
- a transmission device (43) for transmission of the information detected by the detectors (1) in the detector unit (2) to an evaluation device (44); and
- an evaluation device (44) which is designed to calculate a measurement result from a measurement object (42) through which the X-ray radiation (40) has passed on the basis of the information detected by the detectors (1) in the detector unit (2) ;
**characterized**
**in that** the individual detectors (1) in the detector unit (2) are designed to detect both the intensity and the quantum energy of the individual X-ray quanta in the received X-ray radiation (40), and, for each measurement period, to emit a spectrum which, in addition to information about the number of X-ray quanta of medium quantum energy received in each measurement period, and hence the intensity, also contains information about the respective quantum energy in the X-ray quanta, and thus the spectrum of the received X-ray radiation; and
**in that** the evaluation device (44) is also designed to calculate the measurement result from the measurement object (42) on the basis of the information detected by the detectors (1) relating to the intensity and quantum energy of the individual X-ray quanta in the received X-ray radiation (40), taking into account the intensity and the spectrum of the X-ray radiation (40) emitted from the radiation source (41).

2. Computer tomograph according to Claim 1,
**characterized**
**in that** the detectors (1) in the detector unit (2) have a large number of parallel-connected comparators (131, 132, 133), each having a threshold value, and
**in that** each comparator (131, 132, 133) has an associated counter (151, 152, 153), and the comparators (131, 132, 133) are designed to increment the respectively associated counter (151, 152, 153) by one unit when the quantum energy of an X-ray quantum in the received X-ray radiation (40) exceeds the threshold value of the respective comparator (131, 132, 133).

3. Computer tomograph according to Claim 2,
**characterized**
**in that** the threshold values of the comparators (131, 132, 133) are freely variable.

4. Computer tomograph according to Claim 2 or 3,
**characterized**
**in that** the detectors (1) in the detector unit (2) have a large number of pulse logic devices (141, 142, 143), with one pulse logic device (141, 142, 143) in each case being connected downstream from the respective comparators (131, 132, 133), and the respective counters (151, 152, 153) being connected upstream, and the pulse logic devices (141, 142, 143) providing time normalization of the output signals from the comparators (131, 132, 133).

5. Computer tomograph according to one of the preceding claims,
**characterized**
**in that** the detectors (1) in the detector unit (2) have a receiving area (3) for the X-ray radiation (40), which receiving area (3) is formed from gadoliniumoxysulfide ceramic, bismuth germanium oxide or lutetium oxyorthosilicate.

6. Computer tomograph according to one of Claims 1 to 4,
**characterized**
**in that** the detectors (1) in the detector unit (2) have a direct-conversion receiving area (3) for the X-ray radiation (40), which receiving area (3) is formed from cadmium zinc telluride or cadmium telluride.

7. Method for verification of X-ray radiation by means of a computer tomograph which has a detector unit (2) comprising a large number of detectors (1), having the following steps:
- detection of the number of X-ray quanta whose quantum energy exceeds a predetermined threshold value of the X-ray radiation (40) received by means of the individual detectors (1) in the detector unit (2);
- transmission of the information detected by means of the detectors (1) in the detector unit (2) to an evaluation device (44); and
- calculation of a measurement result from a measurement object (42) through which the X-ray radiation (40) has passed by means of the evaluation device (44) on the basis of the information detected by the detectors (1) in the detector unit (2) ;
**characterized**
**in that** both the intensity and the quantum energy of the individual X-ray quanta in the X-ray radiation (40) received by means of the individual detectors (1) in the detector unit is detected,
**in that** the individual detectors (1) in the detector unit (2) emit, for each measurement period, a spectrum which, in addition to information about the number of X-ray quanta of medium quantum energy received in each measurement period, and hence the intensity, also contains information about the respective quantum energy of the X-ray quanta, and thus the spectrum of the received X-ray radiation, and
**in that** the measurement result from the measurement object (42) is calculated by means of the evaluation device (44) on the basis of the information detected by the detectors (1) relating to the intensity and quantum energy of the individual X-ray quanta in the received X-ray radiation (40), taking into account the intensity and the spectrum of the X-ray radiation (40) emitted from a radiation source (41).

8. Method for verification of radiation according to Claim 7,
**characterized**
**in that** the detection of the X-ray quanta which are received by means of the detector (1) in the detector unit (2) comprises the following steps:
- detection of a signal which is produced in the detector (1) as a consequence of a received X-ray quantum, whose signal level is proportional to the quantum energy in the received X-ray quantum;
- comparison of the signal level with a large number of predetermined threshold values;
- incrementation of a counter (151, 152, 153), which is in each case associated with one range between two adjacent threshold values, by one unit when the signal level of the signal is in the range between the two adjacent threshold values.

9. Method for verification of radiation according to Claim 7,
**characterized**
**in that** the detection of the X-ray quanta which are received by means of the detector (1) in the detector unit (4) comprises the following steps:
- detection of a signal which is produced in the detector (1) as a consequence of a received X-ray quantum, whose signal level is proportional to the quantum energy in the received X-ray quantum;
- comparison of the signal level with a large number of predetermined threshold values;
- incrementation of counters (151, 152, 153), which are each associated with one threshold value, by one unit when the signal level of the signal exceeds the respective threshold value.

10. Method for verification of radiation according to Claim 8 or 9,
**characterized**
**in that** a signal which is produced in the detector (1) as a consequence of a received X-ray quantum is rejected if the determined signal level of the signal is lower than a lowest threshold value.

11. Method for verification of radiation according to Claim 8, 9 or 10,
**characterized**
**in that** the threshold values are freely variable.

## Revendications

1. Tomodensitomètre comportant :
- une source de rayonnement (41) pour émettre un rayonnement X (40) ayant une intensité prescrite et ayant un spectre prescrit ;
- une unité de détection (2) constituée de plusieurs détecteurs (1) pour déceler un rayonnement X (40), les différents détecteurs (1) de l'unité de détection (2) étant conçus pour recevoir des quanta incidents du rayonnement X (40) et pour détecter le rayonnement X reçu (40) en ce qui concerne le nombre de quanta de rayonnement X dont l'énergie quantique dépasse une valeur de seuil prescrite ;
- un dispositif de transmission (43) pour transmettre les informations acquises par les détecteurs (1) de l'unité de détection (2) à un dispositif d'évaluation (44) ; et
- un dispositif d'évaluation (44) qui est conçu pour calculer à l'aide des informations acquises par les détecteurs (1) de l'unité de détection (2) un résultat de mesure d'un objet de mesure (42) qui est traversé par le rayonnement X (40) ;
**caractérisé par le fait que**
les différents détecteurs (1) de l'unité de détection (2) sont conçus pour détecter le rayonnement X reçu (40) aussi bien en ce qui concerne son intensité qu'en ce qui concerne l'énergie quantique des quanta individuels du rayonnement X reçu (40) et pour délivrer pour chaque période de mesure un spectre qui contient, outre une information sur le nombre des quanta de rayonnement X d'énergie quantique moyenne reçus par période de mesure et donc outre l'intensité, une information sur l'énergie quantique respective des quanta de rayonnement X et donc sur le spectre du rayonnement X reçu (40) ; et
le dispositif d'évaluation (44) est aussi conçu pour calculer le résultat de mesure de l'objet de mesure (42) à l'aide des informations acquises par les détecteurs (1), à savoir intensité et énergie quantique des quanta individuels du rayonnement X reçu (40), en tenant compte de l'intensité et du spectre du rayonnement X (40) émis par la source de rayonnement (41).

2. Tomodensitomètre selon la revendication 1,
**caractérisé par le fait que**
les détecteurs (1) de l'unité de détection (2) comportent plusieurs comparateurs (131, 132, 133) branchés en parallèle et ayant chacun une valeur de seuil, et
un compteur (151, 152, 153) est associé à chaque comparateur (131, 132, 133) et les comparateurs (131, 132, 133) sont conçus pour augmenter d'une unité le compteur respectivement associé (151, 152, 153) lorsque l'énergie quantique d'un quantum du rayonnement X reçu (40) dépasse la valeur de seuil du comparateur respectif (131, 132, 133).

3. Tomodensitomètre selon la revendication 2,
**caractérisé par le fait que** les valeurs de seuil des comparateurs (131, 132, 133) sont réglables librement.

4. Tomodensitomètre selon la revendication 2 ou 3,
**caractérisé par le fait que** les détecteurs (1) de l'unité de détection (2) comportent plusieurs logiques à impulsions (141, 142, 143), une logique à impulsions (141, 142, 143) étant branchée à chaque fois du côté aval des comparateurs respectifs (131, 132, 133) et du côté amont des compteurs respectifs (151, 152, 153) et les logiques à impulsions (141, 142, 143) provoquant une normalisation temporelle des signaux de sortie des comparateurs (131, 132, 133).

5. Tomodensitomètre selon l'une des revendications précédentes,
**caractérisé par le fait que** les détecteurs (1) de l'unité de détection (2) comportent une surface de réception (3) pour le rayonnement X (40) qui est formée de céramique d'oxysulfure de gadolinium, d'oxyde de bismuth et germanium ou d'oxyorthosilicate de lutécium.

6. Tomodensitomètre selon l'une des revendications 1 à 4,
**caractérisé par le fait que** les détecteurs (1) de l'unité de détection (2) comportent une surface de réception à conversion directe (3) pour le rayonnement X (40) qui est formée de tellurure de cadmium et zinc ou de tellurure de cadmium.

7. Procédé pour déceler un rayonnement X au moyen d'un tomodensitomètre comportant une unité de détection (2) constituée de plusieurs détecteurs (1),
ayant les étapes suivantes :
- détection du rayonnement X (40) reçu au moyen des différents détecteurs (1) de l'unité de détection (2) en ce qui concerne le nombre de quanta de rayonnement X dont l'énergie quantique dépasse une valeur de seuil prescrite ;
- transmission des informations acquises au moyen des détecteurs (1) de l'unité de détection (2) à une unité d'évaluation (44) ; et
- calcul d'un résultat de mesure d'un objet de mesure (42) qui est traversé par le rayonnement X (40), au moyen de l'unité d'évaluation (44) et à l'aide des informations acquises par les détecteurs (1) de l'unité de détection (2) ;
**caractérisé par le fait que**
le rayonnement X reçu (40) au moyen des détecteurs (1) de l'unité de détection (2) est détecté aussi bien en ce qui concerne son intensité qu'en ce qui concerne l'énergie quantique des quanta individuels du rayonnement X reçu (40) ;
les différents détecteurs (1) de l'unité de détection (2) délivrent pour chaque période de mesure un spectre qui contient, outre une information sur le nombre des quanta de rayonnement X d'énergie quantique moyenne reçus par période de mesure et donc outre l'intensité, une information sur l'énergie quantique respective des quanta de rayonnement X et donc sur le spectre du rayonnement X reçu (40), et
le calcul du résultat de mesure de l'objet de mesure (42) s'effectue au moyen du dispositif d'évaluation (44) et à l'aide des informations acquises par les détecteurs (1), à savoir intensité et énergie quantique des quanta individuels du rayonnement X reçu (40), en tenant compte de l'intensité et du spectre du rayonnement X (40) émis par la source de rayonnement (41).

8. Procédé pour déceler un rayonnement selon la revendication 7,
**caractérisé par le fait que** la détection des quanta de rayonnement X reçu au moyen du détecteur (1) de l'unité de détection (2) comporte les étapes suivantes :
- détection d'un signal qui est produit dans le détecteur (1) suite à un quantum de rayonnement X reçu et dont la hauteur est proportionnelle à l'énergie quantique du quantum de rayonnement X reçu ;
- comparaison de la hauteur de signal à plusieurs valeurs de seuil prescrites ;
- augmentation, d'une unité, d'un compteur (151, 152, 153) associé respectivement à une plage entre deux valeurs de seuil voisines lorsque la hauteur du signal se trouve dans la plage entre les deux valeurs de seuil voisines.

9. Procédé pour déceler un rayonnement selon la revendication 7,
**caractérisé par le fait que** la détection des quanta de rayonnement X reçu au moyen du détecteur (1) de l'unité de détection (4) comporte les étapes suivantes :
- détection d'un signal qui est produit dans le détecteur (1) suite à un quantum de rayonnement X reçu et dont la hauteur est proportionnelle à l'énergie quantique du quantum de rayonnement X reçu ;
- comparaison de la hauteur de signal à plusieurs valeurs de seuil prescrites ;
- augmentation, d'une unité, de compteurs (151, 152, 153) associés respectivement à une valeur de seuil lorsque la hauteur du signal dépasse la valeur de seuil respective.

10. Procédé pour déceler un rayonnement selon la revendication 8 ou 9,
**caractérisé par le fait qu'**un signal produit dans le détecteur (1) suite à un quantum de rayonnement X reçu est rejeté lorsque la hauteur déterminée du signal est inférieure à une valeur de seuil minimale.

11. Procédé pour déceler un rayonnement selon la revendication 8, 9 ou 10,
**caractérisé par le fait que** les valeurs de seuil sont librement réglables.
